# EUROPEAN PATENT SPECIFICATION

(11) **EP 4 342 462 B1**
(45) Date of publication and mention of the grant of the patent: **06.05.2026**
(21) Application number: 22215846.1
(22) Date of filing: 22.12.2022
(51) Int. Cl.: A61K 31/167, A61P 17/06, C12N 15/00, C12N 9/48, C07K 14/54, C07K 14/72

(54) **ACEBUTOLOL HYDROCHLORIDE FOR USE IN TREATING PSORIASIS**
ACEBUTOLHYDROCHLORID ZUR VERWENDUNG BEI DER BEHANDLUNG VON PSORIASIS
CHLORHYDRATE D'ACÉBUTOLOL POUR UNE UTILISATION DANS LE TRAITEMENT DU PSORIASIS

(30) Priority: 26.09.2022 CN 202211174329
(43) Date of publication of application: 27.03.2024
(73) Proprietor: Sun, Liangdan, Hefei City, Anhui 230022 (CN)
(72) Inventor: SUN, Liangdan, Hefei City, Anhui (CN); ZHEN, Qi, Hefei City (CN); CHEN, Weiwei, Hefei City (CN); WANG, Yirui, Hefei City (CN); LI, Zhuo, Hefei City (CN)
(74) Representative: Herrero & Asociados, S.L.

(56) References cited:
- AZZOUZ BRAHIM ET AL: "Psoriasis risk after beta-blocker exposure: Description of a pharmacovigilance signal", vol. 88, no. 8, 6 April 2022 (2022-04-06), GB, pages 3813 - 3818, XP093076704, ISSN: 0306-5251, Retrieved from the Internet <URL:https://onlinelibrary.wiley.com/doi/full-xml/10.1111/bcp.15330> DOI: 10.1111/bcp.15330
- KABBUR HANUMANTHAPPA BASAVARAJ ET AL: "The role of drugs in the induction and/or exacerbation of psoriasis", INTERNATIONAL JOURNAL OF DERMATOLOGY, WILEY-BLACKWELL PUBLISHING LTD, UK, vol. 49, no. 12, 23 November 2010 (2010-11-23), pages 1351 - 1361, XP071189074, ISSN: 0011-9059, DOI: 10.1111/J.1365-4632.2010.04570.X
- GOLD M H ET AL: "Beta-blocking drugs and psoriasis", JOURNAL OF THE AMERICAN ACADEMY OF DERMATOLOGY, MOSBY, INC, US, vol. 19, no. 5, 1 November 1988 (1988-11-01), pages 837 - 841, XP025588090, ISSN: 0190-9622, [retrieved on 19881101]
- ABEL E A ET AL: "Drugs in exacerbation of psoriasis", JOURNAL OF THE AMERICAN ACADEMY OF DERMATOLOGY, MOSBY, INC, US, vol. 15, no. 5, 1 November 1986 (1986-11-01), pages 1007 - 1022, XP024559653, ISSN: 0190-9622, [retrieved on 19861101], DOI: 10.1016/S0190-9622(86)70265-X
- JUAN FRANCISCO MIR-BONAFÉ ET AL: "Psoriasis precipitated by timolol eye drops. A series of eight cases", AUSTRALASIAN JOURNAL OF DERMATOLOGY, AUSTRALIAN COLLEGE OF DERMATOLOGISTS, SYDNEY, AU, vol. 61, no. 1, 6 September 2019 (2019-09-06), XP071078478, ISSN: 0004-8380, DOI: 10.1111/AJD.13155
- BARTLEY AKEEM ET AL: "Increased Abundance of Lactobacillales in the Colon of Beta-Adrenergic Receptor Knock Out Mouse Is Associated With Increased Gut Bacterial Production of Short Chain Fatty Acids and Reduced IL17 Expression in Circulating CD4+ Immune Cells", FRONTIERS IN PHYSIOLOGY, vol. 9, 13 November 2018 (2018-11-13), CH, XP093115868, ISSN: 1664-042X, DOI: 10.3389/fphys.2018.01593
- MARTYNIUK CHRISTOPHER J. ET AL: "Genetic ablation of bone marrow beta-adrenergic receptors in mice modulates miRNA-transcriptome networks of neuroinflammation in the paraventricular nucleus", PHYSIOLOGICAL GENOMICS, vol. 52, no. 4, 24 February 2020 (2020-02-24), US, pages 169 - 177, XP093115878, ISSN: 1094-8341, DOI: 10.1152/physiolgenomics.00001.2020
- JOYCE WILLIAM ET AL: "Regulation of heart rate following genetic deletion of the ß1 adrenergic receptor in larval zebrafish", vol. 235, no. 4, 16 June 2022 (2022-06-16), GB, XP093116088, ISSN: 1748-1708, Retrieved from the Internet <URL:https://onlinelibrary.wiley.com/doi/full-xml/10.1111/apha.13849> DOI: 10.1111/apha.13849
- FU ET AL: "Down-regulation of @b"1-adrenoceptors gene expression by short interfering RNA impairs the memory retrieval in the basolateral amygdala of rats", NEUROSCIENCE LETTERS, ELSEVIER, AMSTERDAM, NL, vol. 428, no. 2-3, 6 November 2007 (2007-11-06), pages 77 - 81, XP022332507, ISSN: 0304-3940, DOI: 10.1016/J.NEULET.2007.09.040
- QI CONG ET AL: "Gamma Delta T Cells and Their Pathogenic Role in Psoriasis", vol. 12, 25 February 2021 (2021-02-25), Lausanne, CH, XP093117005, ISSN: 1664-3224, Retrieved from the Internet <URL:https://www.ncbi.nlm.nih.gov/pmc/articles/PMC7959710/pdf/fimmu-12-627139.pdf> DOI: 10.3389/fimmu.2021.627139
- GREINER ET AL: "Reversible transverse overcurvature of the nails (pincer nails) after treatment with a @b-blocker", JOURNAL OF THE AMERICAN ACADEMY OF DERMATOLOGY, MOSBY, INC, US, vol. 39, no. 3, 1 September 1998 (1998-09-01), pages 486 - 487, XP005697903, ISSN: 0190-9622, DOI: 10.1016/S0190-9622(98)70329-9

## Description

### TECHNICAL FIELD

The invention relates to the technical field of dermatologic drugs, and more particularly to a use/application of an adrenoceptor beta 1 (ADRB1) active inhibitor in preparing a preparation or as a preparation for treating psoriasis.

### BACKGROUND

Psoriasis is a chronic inflammatory skin disease characterized by infiltration of immune cells, epidermal hyperplasia, and abnormal differentiation of keratinocytes. Previous studies have found that keratinocytes and immune cells interact to produce cytokine networks, especially interleukin 17 (IL-17) family and its subsequent signal cascades, which drive the development of psoriasis. Psychological and neurological factors are one of inducements of pathogenesis and aggravation of psoriasis, and neuroendocrine is one of important functions of a skin peripheral nervous system. Psychological factors, as a stressor, mainly cause the activation of two major neuroendocrine systems, namely, the hypothalamic-pituitary-adrenal (HPA) axis and the sympathetic-adrenal medullary system (SAM), and cause the cascade reaction of neuroendocrine hormones and the release of proinflammatory cytokines. As psoriasis is related to the increased activity of a variety of proinflammatory cytokines, thereby further inducing or aggravating psoriasis.

At present, studies have shown that IL17A/F is an important cytokine of effector cells of psoriasis, and the clinical use of biological agents targeting IL17A/F has a good therapeutic effect on psoriasis. However, the related biological agents have a high price and need to be used continuously in the late stage, so that the treatment cost is high and psoriasis cannot be eradicated.

Therefore, a safe and effective preparation for treating psoriasis is necessary.

B. Azzouz et al. (British Journal of Clinical Pharmacology, 2022, vol. 88(8), pages 3813-3818, DOI: 10.1111[bcp.15330) provides a restrospective analysis of psoriasis risk after beta-blocker exposure.

K. H. Basavaraj et al. (International Journal of Dermatology, 2010, vol. 49 (12), pages 1351-1361, DOI: 10.1111/J.1365-4632.2010.04570.X) discloses that psoriasis is a common skin disorder; knowledge of the factors that may induce, trigger, or exacerbate the disease is of primary importance in clinical practice. Drug intake is a major concern in this respect, as new drugs are constantly being added to the list of factors that may influence the course of this disease. Drug ingestion may result in exacerbation of pre-existing psoriasis, in induction of psoriatic lesions on clinically uninvolved skin in patients with psoriasis, or in precipitation of the disease in persons without family history of psoriasis or in predisposed individuals. In view of their relationship to drug-provoked psoriasis, therapeutic agents may be classified as drugs with strong evidence for a causal relationship to psoriasis, drugs about which there are considerable but insufficient data to support the induction or aggravation of the disease, and drugs that are occasionally reported to be associated with aggravation or induction. This review focuses on the most common causative agents for drug-induced, drug-triggered, or drug-aggravated psoriasis, such as β-blockers, lithium, synthetic antimalarial drugs, nonsteroidal anti-inflammatory agents, and tetracyclines, and the mechanisms of action of these drugs in the pathogenesis of psoriasis.

### SUMMARY

In view of the above problems, a purpose of the invention is to provide an adrenoceptor beta 1 (ADRB 1) active inhibitor for use in a method for treating psoriasis. The ADRB1 activity inhibitor acebutolol hydrochloride, abbreviated ACE, can effectively inhibit the increase of norepinephrine (NE, also referred to as noradrenaline) caused by abnormal activation of skin sympathetic nerve, and stimulate the secretion of interleukin 17 (IL-17) of gamma delta (γδ) T cells (also referred to as T lymphocytes), thereby reducing the IL-17-mediated immune response at skin lesions in psoriasis and achieving the effect of treating psoriasis.

In order to achieve the above purpose, the invention may adopt technical solutions as follows.

In an aspect, the invention provides acebutolol hydrochloride for use in a method for treating psoriasis. Specifically, in the invention, IL-17-producing γδT cells are activated by ADRB 1, which indicates that psychological and neurological factors can be used as inducements of pathogenesis and aggravating of psoriasis, provides a potential target for treating psoriasis, and provides help for research and development of drugs for treating psoriasis. It should be noted that an ADRB1 activity inhibitor is known in the art to be a reagent that can inhibit ADRB1 activity, which may be a chemical reagent, such as acebutolol hydrochloride (ACE); and may also be a biological reagent, such as clustered regularly interspaced palindromic repeats-associated protein 9 (CRISPR-Cas9) reagent. According to the present invention, the ADRB1 activity inhibitor for use in the treatment of psoriasis is ACE.

In an embodiment, ACE can inhibit abnormal activation of skin sympathetic nerve.

In an embodiment, ACE can inhibit increase of norepinephrine caused by abnormal activation of skin sympathetic nerve.

In an embodiment, ACE can inhibit the secretion of interleukin 17 (IL-17) by δγT cells

It should be noted that in some autoimmune diseases, autoantigen-stimulated inflammation chronically activates the sympathetic nervous system (SNS). SNS dysregulation is accompanied by immune activation caused by aseptic inflammation, resulting in periodic and cascading sympathetic nerve activity (SNA), systemic inflammation, and local immune activation in diseased tissues. In a prospective study on rheumatoid arthritis, researchers found that the SNS dysregulation is a key trigger factor for the pathogenesis and aggravation of the disease, and is a precursor of disease development. In addition, sympathetic neuropathy has also been reported in arthritic joints of rodents with inflammatory arthritis and in the pancreas of rodent models of autoimmune diabetes. Selective loss of sympathetic nerve in the pancreas of humans with type I diabetes has also been reported. In early adjuvant-induced arthritis (AIA), sympathectomy significantly reduces the severity of acute AIA by inhibiting T helper type 1 (Th1) and Th17 type immune responses, indicating that SNS activity mainly increases the severity of acute exacerbation of immune-mediated arthritis. Psoriasis, as a member of autoimmune diseases, also has typical characteristics of autonomic nervous system (ANS) dysregulation, which is manifested as an imbalance in the activity/reactivity imbalance of SNS and parasympathetic nervous system (PaSNS). There are many lymphoid organs in the SNS loop, and sympathetic nerves are also distributed in the skin. The main neurotransmitter of sympathetic neurons is norepinephrine (NE). Norepinephrine is the major catecholamine hormone expressed in skin tissue and the major neurotransmitter released by sympathetic postganglionic neurons. The sympathetic nervous system stress test can lead to a significant increase in NE in blood circulation of patients with psoriasis, indicating the activation and disorder of SNS in psoriasis.

It should be noted that the invention has verified that NE in the blood circulation of psoriatic patients is significantly higher than that of healthy control population, and imiquimod (IMQ) treatment can lead to the increase of NE levels in the skin and blood circulation of mice. In addition, it is determined that IMQ treatment significantly upregulated tyrosine hydroxylase (TH) phosphorylation in mouse skin, TH is the rate-limiting enzyme for catecholamine hormone synthesis, and NE is the major catecholamine hormone expressed in skin tissue. The establishment of psoriasis mouse model based on drug-induced ablation of skin sympathetic nerve and subcutaneous injection of NE demonstrates that skin sympathetic nerve regulates NE secretion through TH activation and promotes the production of IL-17A by γδT cells.

Specifically, catecholamine hormone can affect the balance of helper T cell (Th) subsets. Th cells are a class of T cells whose main surface marker is cluster of differentiation 4 (CD4). Once activated, Th cells differentiate into different subsets of effector/memory cells, such as Th1, Th2 and Th17 cells, which respectively express corresponding marker cytokines interferon-gamma (IFN-γ), IL-4 and IL-17A. Catecholamine hormones affect the systemic immune system response in a "fight or flight" response, which is controlled by the sympathetic nervous system. SNS releases norepinephrine through adrenergic nerves in the region where T cells are located, and T cells express adrenergic receptors on the surface and react with catecholamines.

In the invention, according to results of single-cell sequencing and immunofluorescence of skin lesions of psoriatic patients and skin tissues of mouse models, it is found that beta 1-adrenergic receptor (β1-AR) is specifically expressed in γδT cells in skin tissues. In addition, catecholamine can indirectly affect the differentiation of Th17 cells by acting on adenomatous polyposis coli (APC) gene. Dendritic cells (DCs) provide co-stimulation and cytokines necessary for CD4⁺T cell function and produce IL-23 that is a cytokine necessary for the amplification of Th17, DCs from mice treated with salbutamol, a β2-AR agonist, produce more IL-23, which promotes the differentiation of effector Th17 cells. NE or salbutamol stimulates DCs in mice to induce IL-17A, reducing the production of IFN-γ by CD4 cells. However, relatively little is known about how catecholamine directly affect skin-resident γδT cells.

In addition, in the invention, in order to verify that NE directly acts on γδT17 cells through β1-AR, the mouse models individually smeared with β1-AR agonists and β1-AR antagonists on the skin surface and γδT cell models applied β1-AR agonists and β1-AR antagonists *in vitro* are constructed, which verifies the hypothesis that NE can regulate the expression of IL-17 through binding to β1-AR on the surface of γδT cells.

Moreover, the T cell models applied β1-AR agonists and β1-AR antagonists *in vitro* verifies that the activity of β1-AR is changed by affecting the activation of nuclear factor kappa-B (NF-κB) to regulate the activation of γδT cells, and the change of NF-xB activity in skin tissues is found the mouse models smeared with the β1-AR agonists or the β1-AR antagonists, which indicates that the skin sympathetic nerve mediates the regulation of γδT17 cells through TH-NE-ADRB 1-NF-κB pathway in the pathogenesis of psoriasis.

The present disclosure provides a preparation which includes ACE as an ADRB1 active inhibitor and pharmaceutical excipients. It should be noted that ADRB1 active inhibitor may be mixed with the pharmaceutical excipients to make a preparation suitable for clinical use, for example, the ADRB1 active inhibitor may be dissolved in 5% dimethyl sulfoxide (DMSO)+95% (20% sulfobutylether-β-cyclodextrin abbreviated SBE-β-CD, in saline) to prepare an ointment, and the ointment may be applied on the back of mice. Alternatively, the ADRB1 active inhibitor may be made into an aqueous solution for oral administration. In addition, the ADRB1 active inhibitor may also be used in combination with other medicines for treating psoriasis, and the specific combination may be adjusted according to the specific clinical situation.

In another aspect, the disclosure provides a method for inhibiting IL-17 secretion from γδT cells *in vitro,* including knocking out or knocking down an ADRB1 expression gene in cells *in vitro* (also referred to as isolated cells). It should be noted that, the secretion of IL-17 by γδT cells can be inhibited by knocking out or knocking down the ADRB1 expression gene in cells *in vitro,* and the method of knocking out or knocking down is a basic editing method known in the art, such as CRISPR-Cas system, transcription activator-like effector nucleases (TALEN), or Zinc-finger nuclease (ZFN).

The beneficial effects of the invention are as follows. The ADRB1 activity inhibitor ACE can effectively inhibit the increase of norepinephrine caused by abnormal activation of skin sympathetic nerve, and stimulate the secretion of IL-17 of γδT cells, thereby reducing the IL-17 mediated immune response in skin lesions of psoriatic patients and achieving the effect of treating psoriasis.

### BRIEF DESCRIPTION OF DRAWINGS

FIGS. 1A-1C illustrate secretion levels of neurotransmitters in peripheral blood of healthy controls (HC) and psoriatic patients.
FIGS. 2A-2D illustrate levels of norepinephrine and dopamine in skin and peripheral blood of control mice and psoriatic mice.
FIGS. 3A-3B illustrate total protein levels and phosphorylated active protein levels of tyrosine hydroxylase (TH) in skins of psoriatic patients and psoriatic mice.
FIG. 4 illustrates a flowchart of an experiment for intradermal injection of 6-hydroxydopamine (6-OHDA) and inducing psoriasis-like phenotype in mice by imiquimod (IMQ).
FIGS. 5A-5E illustrate psoriasis-like phenotypes of mice after intradermal injection of 6-OHDA.
FIG. 6 illustrates proportions of interleukin 17A⁺ (IL-17A⁺) γδT cells in skin of mice after intradermal injection of 6-OHDA.
FIGS. 7A-7B illustrate expressions of norepinephrine (NE) in the skin of mice after intradermal injection of 6-OHDA.
FIG. 8 illustrates a flowchart of an experiment for subcutaneous injection of NE and inducing psoriasis-like phenotype in mice by IMQ.
FIGS. 9A-9E illustrate psoriasis-like phenotypes of mice after subcutaneous injection of NE.
FIGS. 10A-10B illustrate proportions of IL-17A⁺ γδT cells in skin of mice after subcutaneous injection of NE.
FIG. 11 illustrates single-cell sequencing analysis of control mice and psoriatic mice.
FIG. 12 illustrates immunohistochemical analysis of beta-1-adrenergic receptors (β1-AR, also referred to as adrenoceptor beta 1 abbreviated ADRB1) of skin of Vaseline^{®} (VAS)-treated mice and IMQ-treated mice.
FIG. 13 illustrates results of β1-AR in the skin of VAS-treated mice and IMQ-treated mice detected by western blot.
FIG. 14 illustrates immunofluorescence (IF) co-localization of β1-AR, IL-17A, γδT cells, αβT cells in the skin of psoriatic mice.
FIGS. 15A-15B illustrate proportions of β1-AR⁺ T cells in the skin of IMQ-treated mice.
FIG. 16 illustrates immunohistochemical analysis of β1-AR in lesional and non-lesional skin of psoriatic patients and skin of healthy controls.
FIG. 17 illustrates results of β1-AR in the lesional and non-lesional skin of psoriatic patients and the skin of healthy controls detected by the western blot.
FIG. 18 illustrates immunofluorescence co-localization of β1-AR, γδT cells, αβT cells in skin lesions of psoriatic patients and the skin of the healthy controls.
FIGS. 19A-19B illustrate proportions of β1-AR⁺ cells in T cells in the skin lesions of psoriatic patients and the skin of the healthy controls .
FIGS. 20A-20B illustrate distribution of β1-AR⁺ cells in T cell subsets.
FIGS. 21A-21E illustrate skin phenotypes of IMQ-treated *Adrb1 +*/*-* mice.
FIG. 22 illustrates a proportion of monocytes of immune cells in back skin of the IMQ-treated *Adrb1 +*/*-* mice detected by flow cytometry.
FIGS. 23A-23C illustrates proportions of IL-17A⁺ dermis γδT cells, IL-17A⁺ epidermis γδT cells, IL-17A⁺ αβT cell of the IMQ-treated *Adrb1 +*/*-* mice detected by flow cytometry.
FIGS. 24A-24E illustrate skin phenotypes of IMQ-induced mice smeared with acebutolol hydrochloride (ACE).
FIGS. 25A-25C illustrate production of IL-17A in the IMQ-induced mice smeared with ACE.
FIGS. 26A-26E illustrate skin phenotypes of IMQ-induced mice smeared with dobutamine hydrochloride (DOB).
FIGS. 27A-27C illustrate production of IL-17A in the IMQ-induced mice smeared with DOB.
FIG. 28 illustrates skin phenotypes of γδT-cell deficient mice (*Tcrd* -/-) smeared with DOB.
FIGS. 29A-29B illustrate proportions of monocytes and neutrophils in skin of T-cell deficient mice (*Tcrd -*/*-*) after subcutaneous injection of DOB.
FIG. 30 illustrates production of IL-17 in αβT cells of the T-cell deficient mice (Tcrd - /-) after subcutaneous injection of DOB.
FIGS. 31A-31B illustrate transcription of IL17A/F in primary γδT cells stimulated by IL-23 and ACE/DOB.
FIG. 32 illustrates activation of nuclear factor kappa-B (NF-κB), protein kinase B (AKT), p38 and extracellular signal-regulated kinase (ERK) in the primary γδT cells stimulated by IL-23 and ACE/DOB.
FIG. 33 illustrates NF-κB phosphorylation in skin of IMQ-induced mice after DOB treatment.
FIG. 34 illustrates NF-κB phosphorylation in skin of IMQ-induced mice after ACE.

### DETAILED DESCRIPTION OF EMBODIMENTS

The present invention is defined by the claims. Any subject-matter falling outside the scope of the claims is provided for information only.

Expressions in the singular include expressions in the plural unless they have a significantly different meaning in the context. As used herein, it should be understood that terms such as "including", "having", and "comprising" are intended to indicate the presence of features, numbers, operations, components, parts, elements, materials, or combinations thereof. The terms of the invention are disclosed in the specification and are not intended to exclude the possibility that one or more other features, numbers, operations, components, parts, elements, materials, or combinations thereof may exist or may be added. As used herein, "/" may be interpreted as "and" or "or", as the case may be.

In order to understand the invention better, the contents of the invention are further illustrated below in combination with specific examples, but the contents of the invention are not limited to the following examples.

In the following examples, experimental procedures involved that are not specifically described are known in the art.

### Example 1-Expression of norepinephrine in psoriatic patients and psoriatic mice

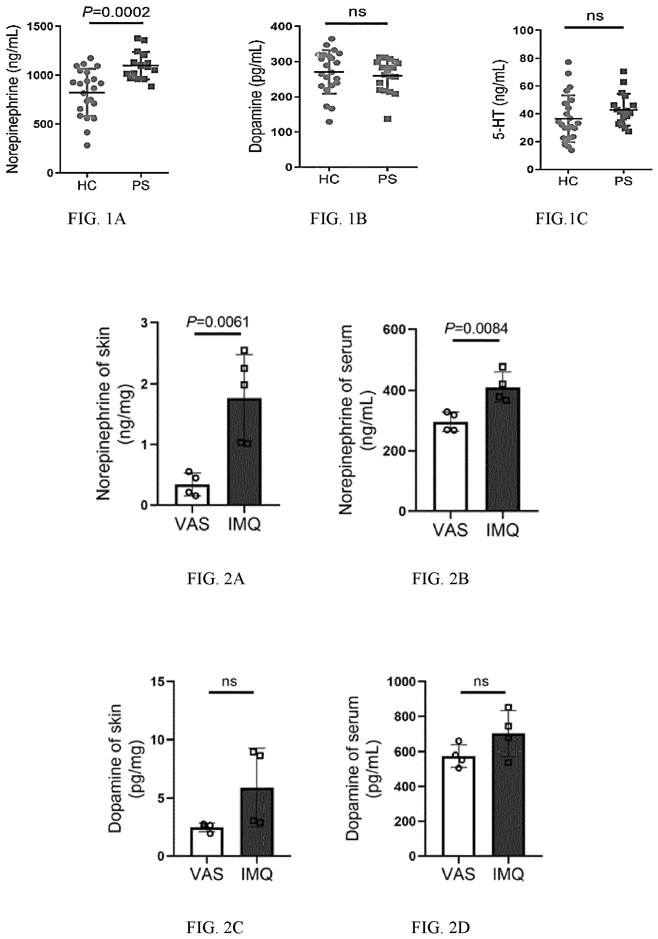

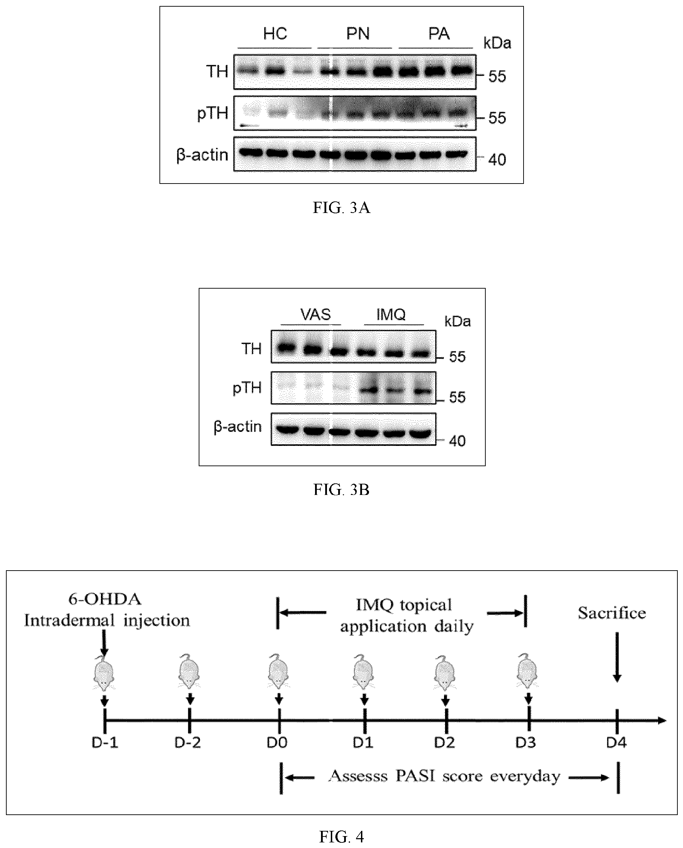

In the example of the invention, it is found by enzyme-linked immunosorbent assay (ELISA) that the norepinephrine (NE) content in serums of psoriatic patients is higher than that of healthy controls, while no significant difference is found in dopamine and 5-hydroxytryptamine (5-HT) (as shown in FIGS. 1A-1C). In addition, the NE content in both skin and serum of psoriatic mice treated with imiquimod (IMQ) is higher than that of control mice treated with VAS, while no difference is found in dopamine (as shown in FIGS. 2A-2D). The existing literature has proved that sympathetic nerve participates in regulating catecholamine biosynthesis by stimulating tyrosine hydroxylase (TH) phosphorylation. Western blot detection shows that the total protein level and phosphorylation of TH in lesional and non-lesional skins of psoriatic patients are higher than those in skins of the healthy controls, and IMQ treatment results in significant up-regulation of TH phosphorylation in mouse skin (as shown in FIGS. 3A-3B).

### Example 2-Construction of psoriasis model with skin sympathetic nerve ablation

In the example of the invention, the sympathectomy of local skin is as follows. 0.6 micrograms (mg) of 6-hydroxydopamine (6-OHDA) are dissolved into 100 microliters (µL) of 0.1% ascorbic acid solution (diluted with 0.9% sterile sodium chloride) to prepare a fresh sympathetic nerve ablation solution. Wild-type mice aged 7-8 weeks are selected to shave their back hair, and the exposed skin area is 2 centimeters (cm) × 2 cm. Then, the mice are intradermally injected with 100 µL fresh sympathetic nerve ablation solution, controls are injected with an injecting control agent (100 µL of 0.1% ascorbic acid solution). The exposed skin of mice is evenly smeared with a IMQ cream or a smearing control agent Vaseline (VAS) after injection for 48 hours.

It has been disclosed in the existing literature that 6-hydroxydopamine (6-OHDA), a neurotoxin that selectively destroys catecholaminergic neurons, is used to remove sympathetic nerves. Intraperitoneal injection of 6-OHDA can reduce IMQ induced ear swelling, but it does not affect the production of IL-17, which is attributed to cardiovascular effects and/or systemic immune dysfunctions caused by systemic sympathectomy.

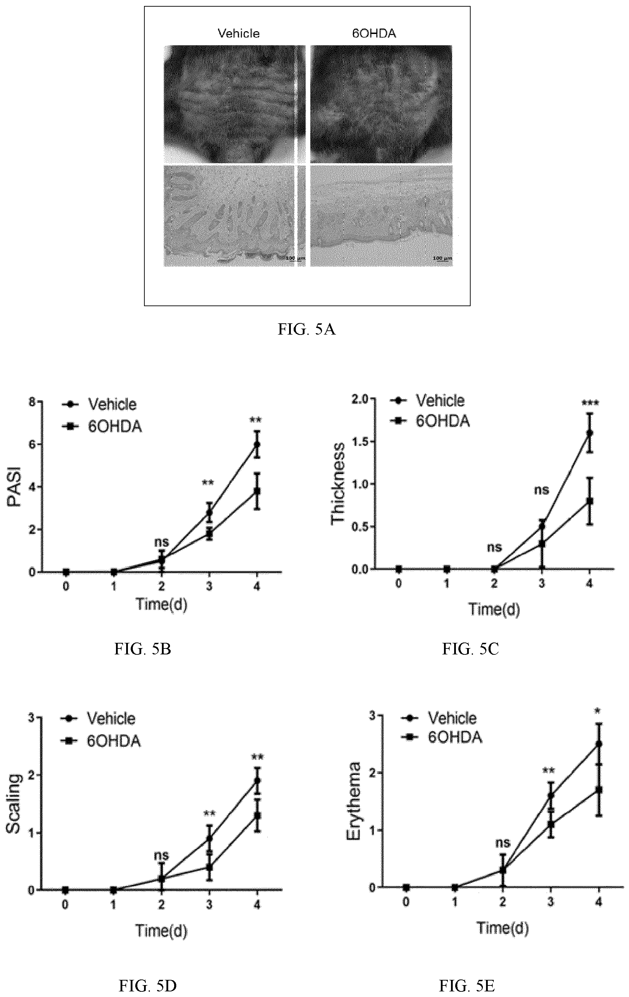

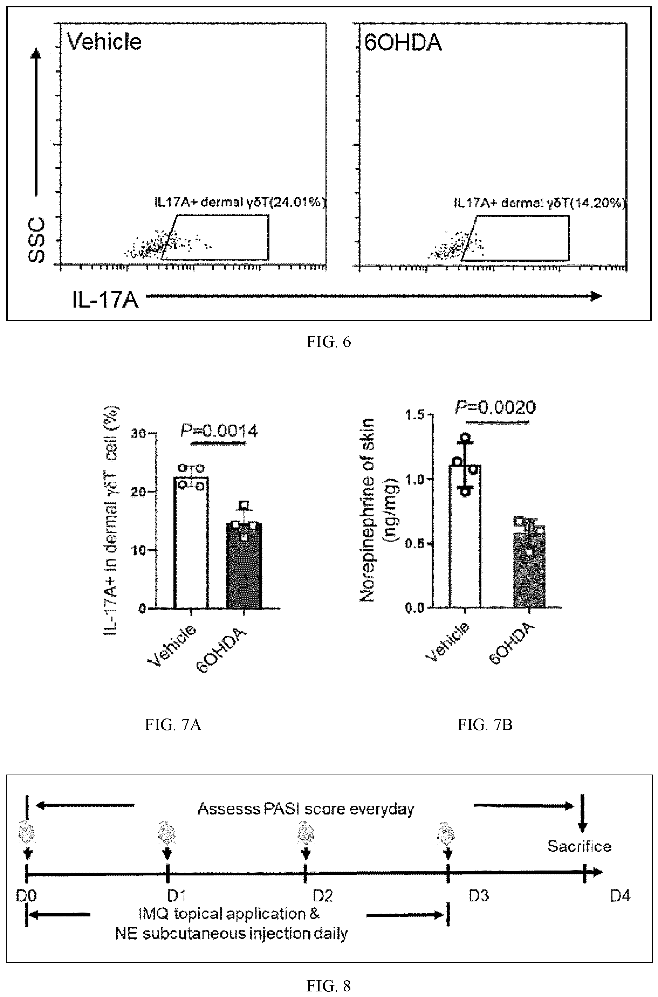

In order to observe the effect of the sympathectomy of local skin on psoriatic mice, 6-OHDA is intradermally injected into mice before IMQ treatment (as shown in FIG. 4). Subsequent studies proved that the removal of skin sympathetic nerve reduces the psoriasis-like phenotype of mice induced by IMQ (as shown in FIGS. 5A-5E). In addition, IL-17A+ γδT cells in sympathetic denervated skin is decreased, indicating that skin sympathetic nerve affects psoriasis-like skin inflammation by regulating IL-17 production by γδT cells (as shown in FIG. 6). The decrease of NE in sympathetic denervated skin indicates that sympathetic nerves may regulate IL-17 production of γδT cells by secreting NE (see FIGS. 7A-7B).

### Example 3-Construction of psoriasis model with norepinephrine (NE) injection

In the example of the invention, the construction of psoriasis model injected with norepinephrine (NE) is as follows. 6 µL of norepinephrine storage solution (10 millimoles per liter, abbreviated mM) is dissolved into 100 µL of 1×phosphate-buffered saline (also referred to as 1×PBS, specifically prepared by a commonly used 1× phosphate buffered saline recipe), and 0.1 micrograms per milliliter (mg/mL) NE diluent is prepared. Wild-type mice aged 7-8 weeks are selected to shave their back hair, and the exposed skin area is 2 cm × 2 cm. Then, the mice are subcutaneously injected with 100 µL NE diluent, and controls are injected with an injecting control agent (6 µL dimethyl sulfoxide, abbreviated DMSO + 100 µL PBS). After the drug is absorbed (the skin mound disappears after injection), the IMQ cream or the smearing control agent VAS is evenly applied on the exposed skin of mice, avoiding the injecting position, and continuously inject and smear for 4 days.

Sympathetic nerves are the main source of NE in the skin, and the decrease of NE in the skin of mice with sympathetic nerve removal further proves that skin sympathetic nerves are the main source of skin NE.

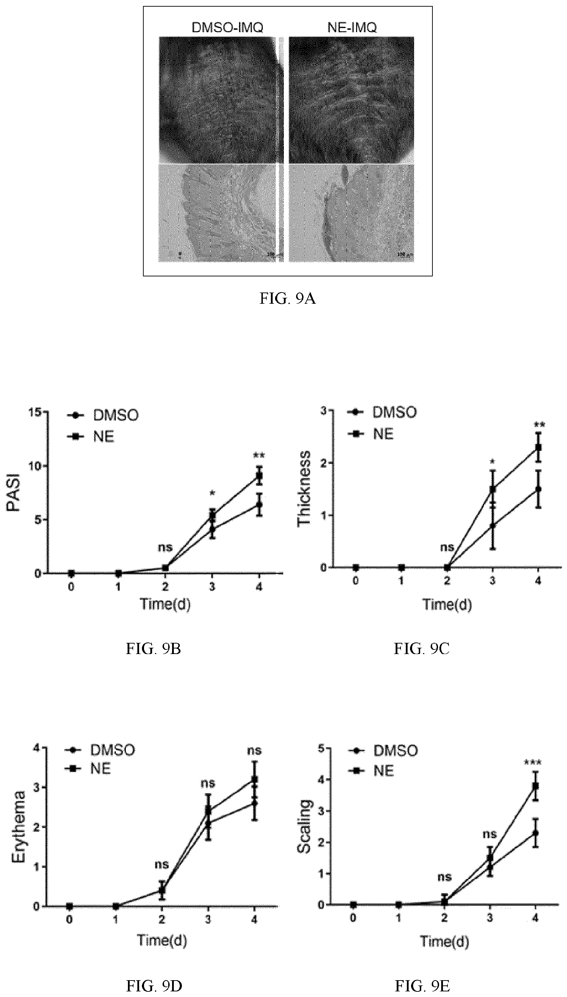

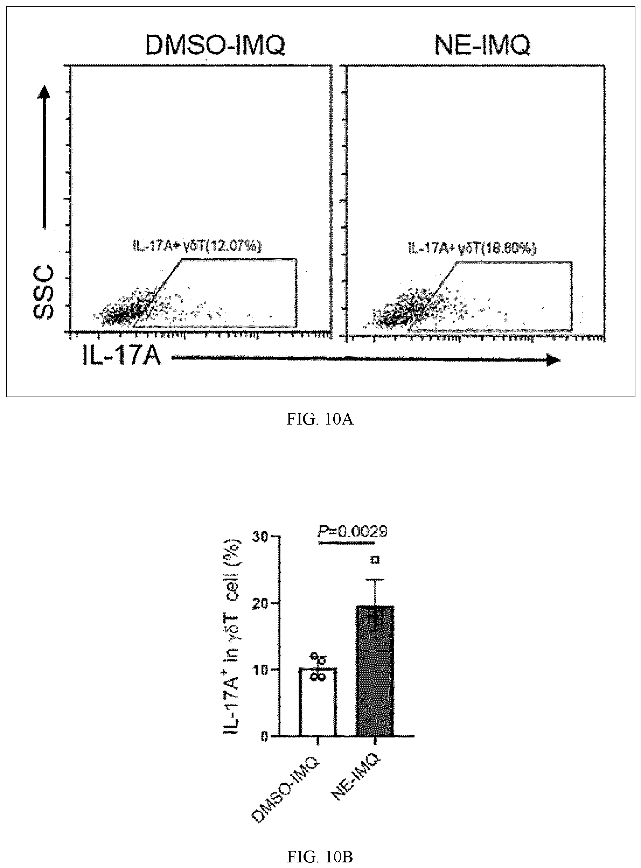

In order to verify whether the secretion of NE in the skin affects the psoriasis-like phenotype of mice, NE is subcutaneously injected while IMQ is smeared on the exposed skin of the back of mice (as shown in FIG. 8). It is found that NE injection exacerbates the psoriasis-like phenotype of mice induced by IMQ (as shown in FIG. 9A-9E), and increases the proportion of IL-17A + γδT cells in the skin of mice (as shown in FIGS. 10A-10B).

### Example 4-Expression pattern of NE receptor in mouse skin

Adrenergic receptor (AR) family is generally divided into α-subtypes and β-subtypes, norepinephrine (NE) is generally considered as a β1-subtype selective adrenergic agonist, which has direct activity on β2-adrenergic receptor only at higher concentrations.

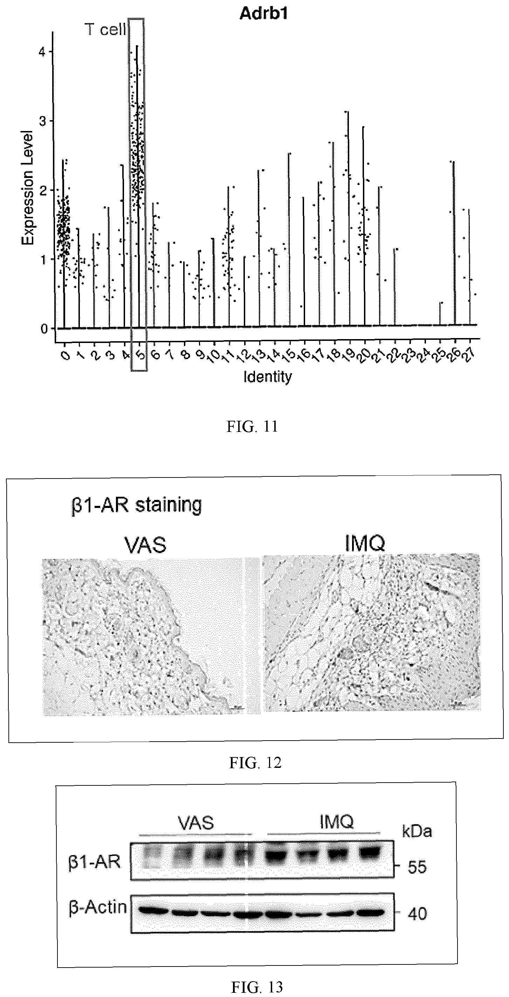

Single-cell sequencing is performed on skin tissues of VAS-treated control mice and IMQ-induced psoriatic mice. The results show that adrenoceptor beta 1 (ADRB1, also referred to as β1-AR) is mainly expressed in T cells of skin tissue (as shown in FIG. 11). The results of immunohistochemistry and western blot show that, β1-AR is expressed on immune cells infiltrating dermis, and its expression in the skin of psoriatic mice is higher than that of control mice (as shown in FIG. 12 and FIG. 13).

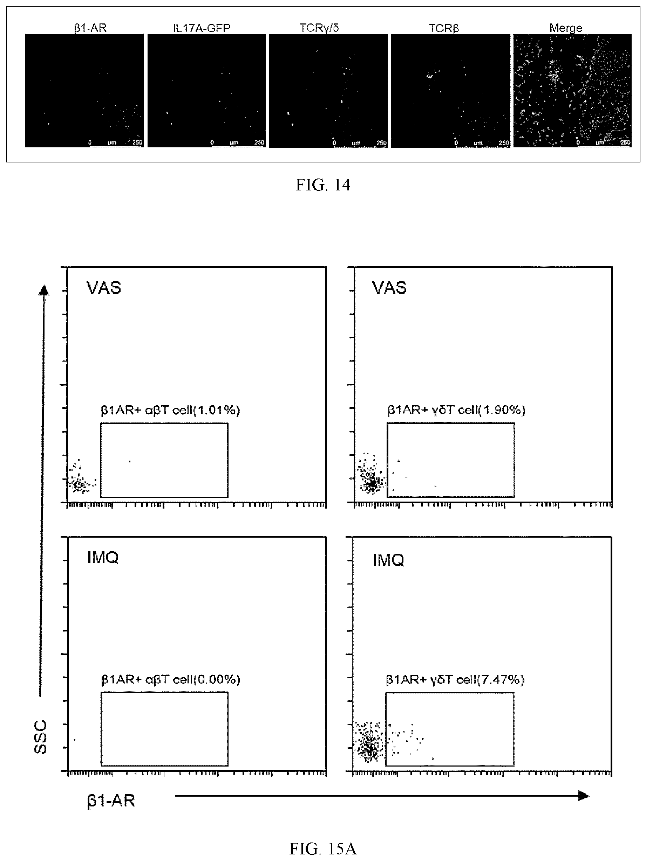

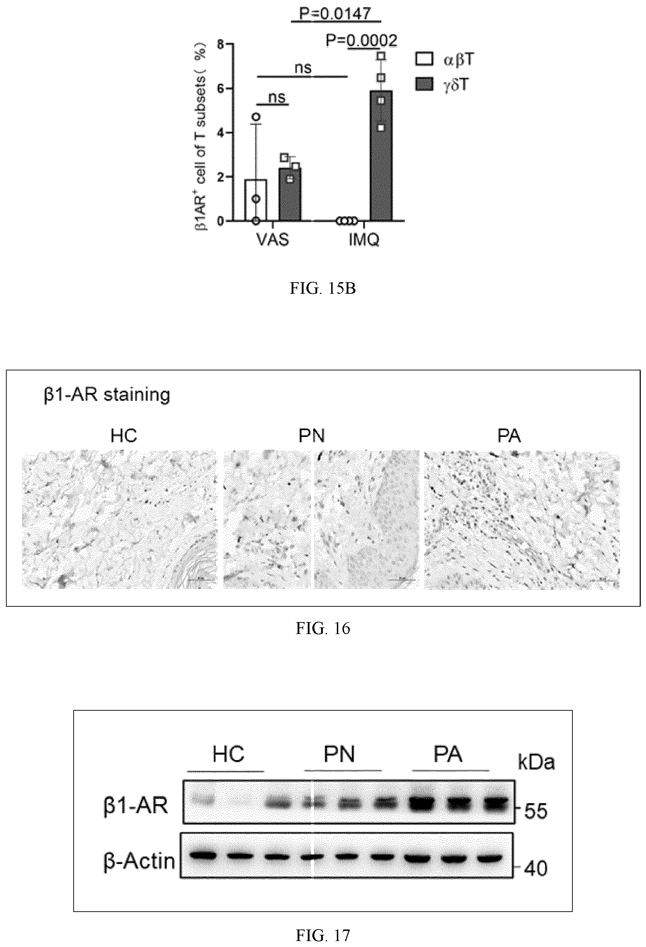

Immunofluorescence and flow cytometry are used to detect β1-AR is mainly expressed in IL-17A-producing γδT cells (also referred to as γδT-17 cells), as shown in FIG. 14. Specifically, there is no significant difference in the proportion of β1-AR⁺ cells between γδT cells and αβT cells in the skin of VAS-treated mice; the proportion of β1-AR⁺ cells (i.e., cells expressed with β1-AR) in γδT cells are significantly higher than that of αβT cells in the skin of IMQ-treated mice; there is no significant difference in the proportion of β1-AR⁺ cells in αβT cells between the skins of VAS-treated mice and IMQ-treated mice; and the proportion of β1-AR⁺ cells in γδT cells in the skin of IMQ-treated mice is significantly higher than that of VAS treated mice (as shown in FIGS. 15A-15B). Therefore, it is concluded that β1-AR is mainly expressed in γδT cells rather than in αβT cells, compared with healthy mice induced by VAS, the expression of β1-AR in γδT cells in the skin of psoriatic mice is significantly increased.

### Example 5-Expression pattern of NE receptor in psoriatic skin

In the example of the invention, the skin lesions of psoriatic patients and the skin of healthy controls are analyzed by immunohistochemistry and western blot. The results show that, β1-AR is expressed on immune cells infiltrating dermis (as shown in FIG. 16), and the expression of β1-AR in psoriatic lesions is higher than in non-lesions, and the expression of β1-AR in non-lesions is higher than in healthy controls (as shown in FIG. 17).

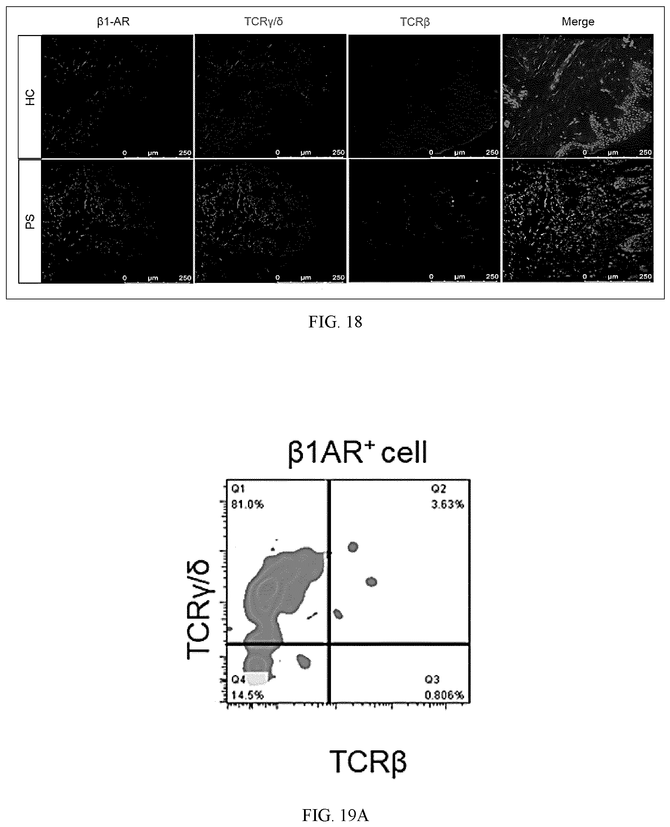

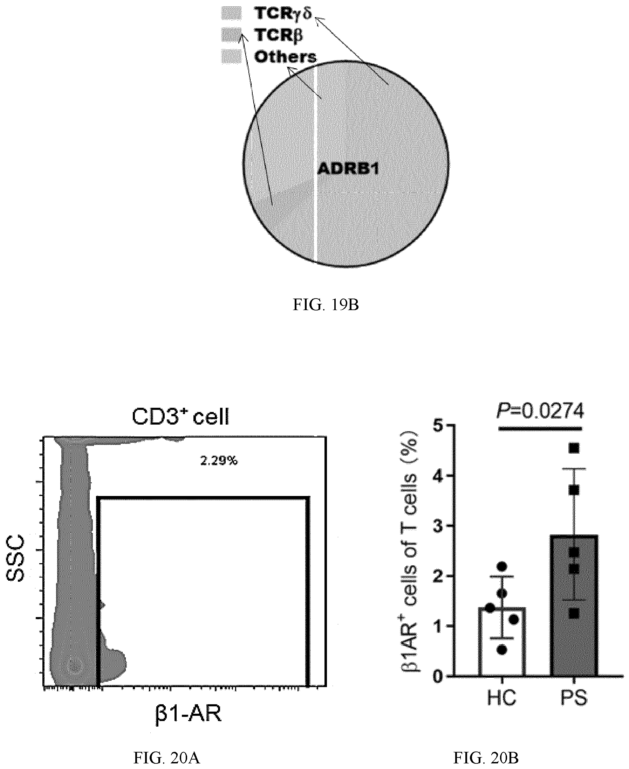

Immunofluorescence and flow cytometry analysis show that, β1-AR is mainly expressed in γδT cells (as shown in FIG. 18 and FIGS. 19A-19B), the proportion of β1-AR⁺ cells in psoriatic patients is higher than that of the healthy controls (as show in FIGS. 20A-20B).

### Example 6-Establishment of IMQ-induced Adrb1 +/- psoriasis mouse model

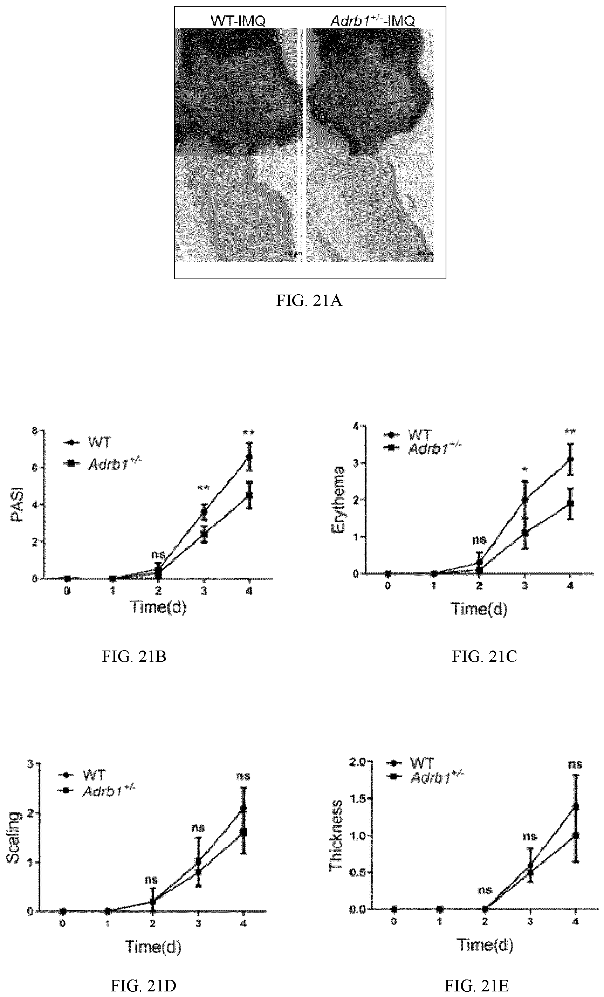

In the example of the invention, in order to study the mechanism of β1-AR in psoriasis, an IMQ-induced *Adrb1* +/- psoriasis mouse model is established (mouse with *Adrb1* lethal knockout). The results show that compared with IMQ-induced wild-type (WT) mice, the psoriasis-like phenotype of *Adrb1 +*/*-* mice is weakened, reflecting that erythema is significantly reduced, but the skin thickness and scaling are not significantly changed (as shown in FIGS. 21A-21E).

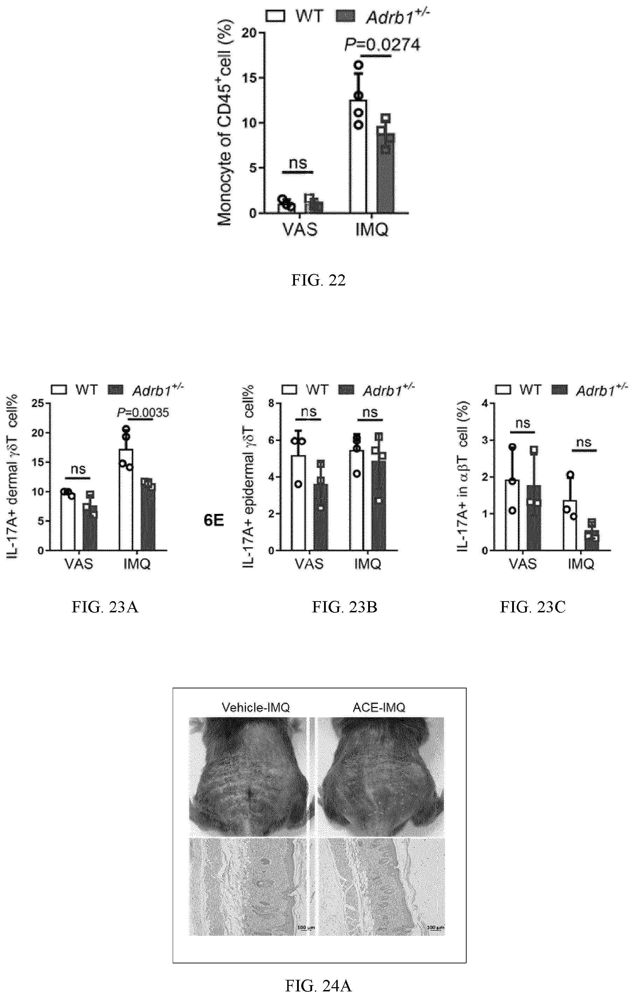

Isolated mouse skin immune cells are grouped by using specific labeled antibodies, in which monocytes and neutrophils are typical inflammatory infiltrating cells in psoriatic lesions. Flow cytometry shows the proportion of monocytes in the back skin of *Adrb1* +/- mice induced by IMQ and the proportion of IL-17A⁺ dermis γδT cells are significantly decreased (as shown in FIG. 22 and FIGS. 23A-23C).

### Example 7-Construction of psoriasis mouse model smeared with β1-AR antagonist

In the example of the invention, the construction of psoriasis mouse model smeared with β1-AR antagonist/agonist is as follows. 5 µL of β1-AR antagonist/agonist storage solution (40 mg/mL, DMSO solvent) is taken and dissolved in 95 µL of 20% sulfobutylether-betacyclodextrin derivative (SBE-β-CD) solution (diluted with sterile sodium chloride) to prepare 2 mg/mL antagonist/agonist diluent. Wild-type mice aged 7-8 weeks are selected to shave their back hair, and the exposed skin area is 2 cm × 2 cm. The mice are applied with 100 µL of the antagonist/agonist diluent, and controls are smeared with a smearing control agent (i.e., 5 µL DMSO + 95 µL 20% SBE- β- CD solution). After the drug is absorbed, the IMQ cream or the control agent VAS is evenly applied on the exposed skin of mice for 4 consecutive days.

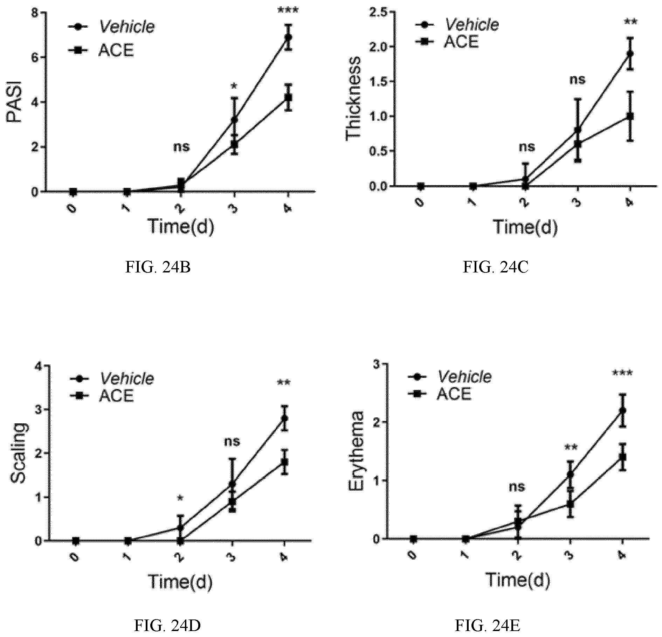

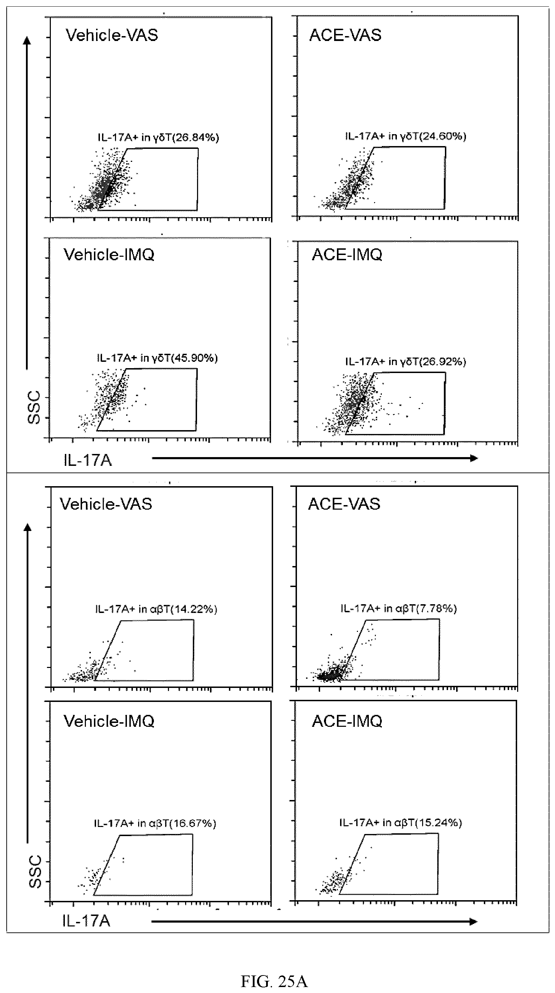

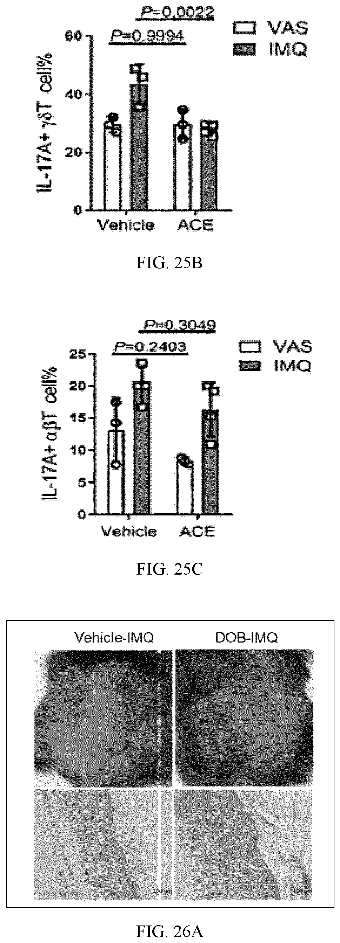

IMQ-induced mice are smeared with acebutolol hydrochloride (ACE, a selective ADRB1 antagonist). The results show that compared with the control group, psoriasis-like phenotype of mice smeared with ACE is reduced (as shown in FIGS. 24A-24E). The proportion of γδT-17 cells in the skin of IMQ-induced mice smeared with ACE is reduced (as shown in FIGS. 25A-25C).

### Example 8-Construction of psoriasis mouse model smeared with β1-AR agonist

In the example of the invention, the construction of psoriasis mouse model of β1-AR antagonist/agonist is the same as that of the embodiment 7.

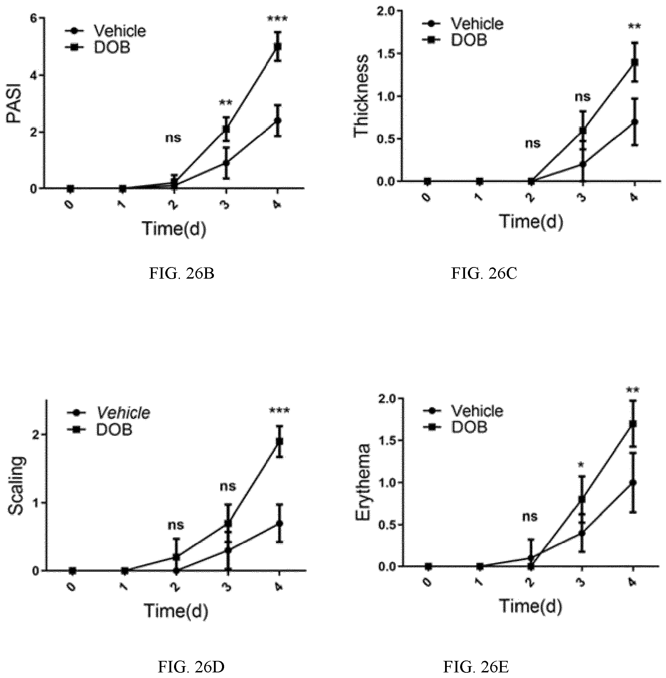

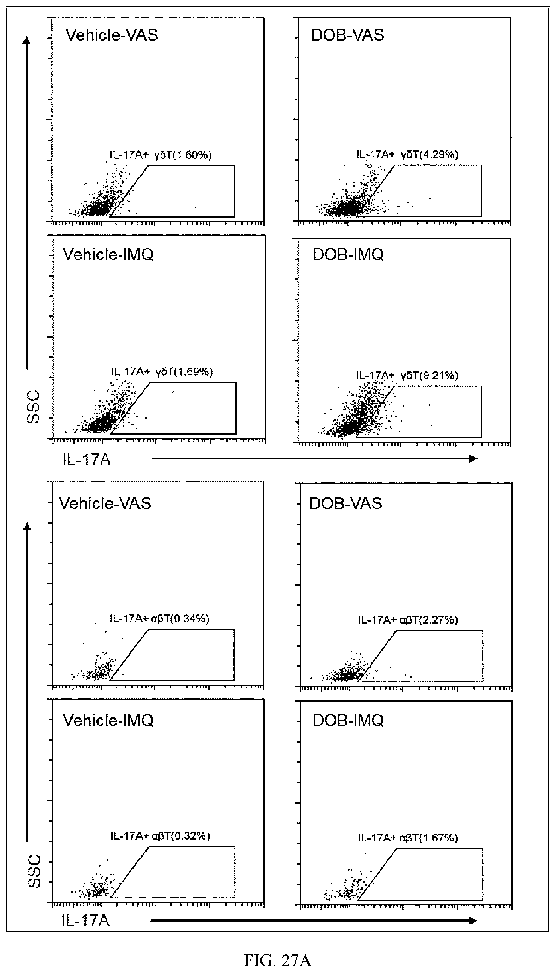

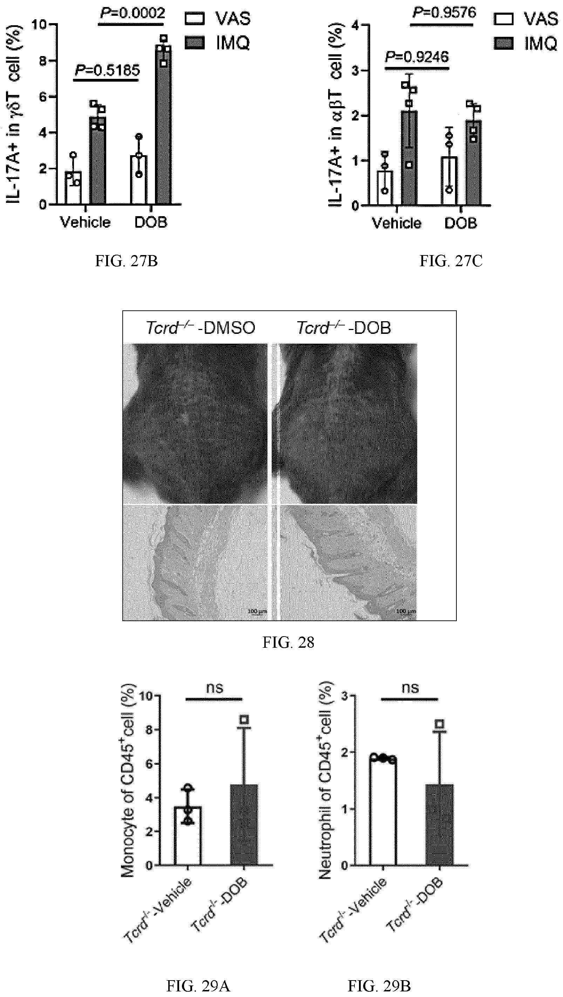

In order to further investigate whether NE directly affects γδT-17 cells through β1-AR, IMQ-induced mice are applied dobutamine hydrochloride (DOB, a selective ADRB1 agonist). The results show that compared with the control group, the psoriasis-like phenotype of mice smeared with DOB is aggravated, including increased back skin thickness, erythema, and scaling (as shown in FIGS. 26A-26E). DOB administration promotes IL-17A production of γδT cells in the skin of IMQ-induced mice (as shown in FIGS. 27A-27C).

### Example 9-Regulation of IL-17 secretion by γδT cells through NE-ADRB1 signaling pathway

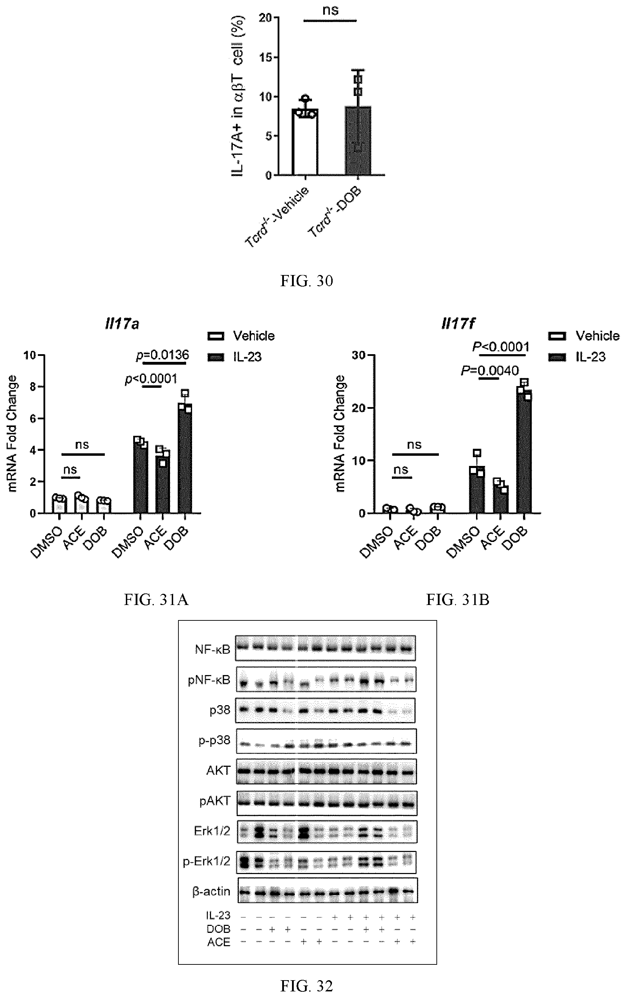

In the example of the invention, DOB is applied to the skin of γδT-cell deficient mice (*Tcrd* -/-) and it is found that due to the lack of γδT cells, the promoting effects of DOB on the psoriasis-like phenotype and inflammatory infiltration are offset (as shown in FIG. 28 and FIGS. 29A-29B). However, DOB has no effect on the IL-17 producing capacity ofαβT cells (as shown in FIG. 30). Combined with the previous results, it is verified that NE in skin is mainly derived from skin sympathetic nerve and mediates the release IL-17 from γδT cells rather than αβT cells through NE-ADRB1 signaling pathway.

### Example 10-Validation of immunoregulation of β1-AR in cell model

In the example of the invention, in order to study how to ADRB1 regulates the inflammatory response of γδT cells, high-purity primary γδT cells are isolated from the spleen of mice by magnetic bead sorting kit, and stimulated with ADRB1 agonist and antagonist respectively.

The results show that high concentration of DOB stimulation can increase the transcription of IL17A and IL17F in primary γδT cells activated by IL-23; in contrast, ACE decreases the transcription of IL17A and IL17F (as shown in FIGS. 31A-31B).

### Example 11-Validation of NE-ADRB1-nuclear factor kappa-B (NF-κB) signaling pathway

In the example of the invention, theDOB stimulation significantly increases NF-κB (P65) phosphorylation in IL-23-activated primary γδT cells, while the ACE treatment inhibits NF-κB (P65) phosphorylation, but they do not affect the activation of members of the mitogen-activated protein kinase (MAPK) family P38, extracellular signal-regulated kinase (ERK), Jun NH2-terminal kinase (JNK) and protein kinase B (AKT) (as shown in FIG. 32). It is indicated that ADRB1 regulates the activation of T cells depending on NF- κB activation.

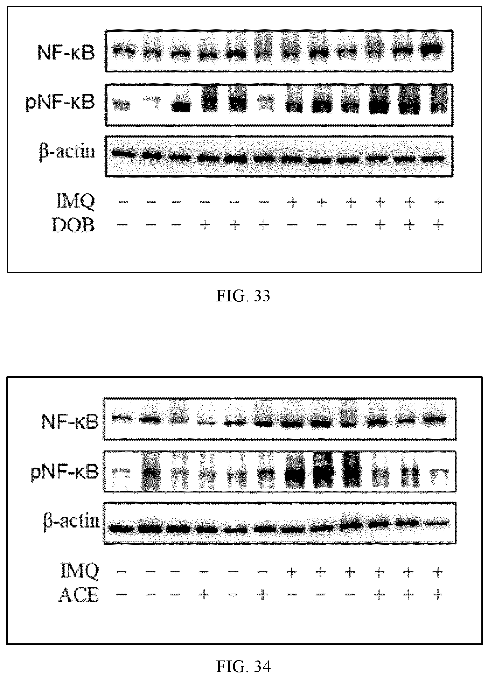

The NF-κB level in the skin of the IMQ-induced psoriatic mice is detected, and it is found that NF-κB phosphorylation is activated by IMQ; DOB treatment enhances activation of NF-κB in the skin of the IMQ-induced psoriatic mice, while ACE treatment shows inhibition for activation of NF-κB in the skin of the IMQ-induced psoriatic mice (as shown in FIG. 33 and FIG. 34).

In summary, sympathetic nerve promotes the production of IL-17 by γδT cells through activating the NE-ADRB1-NF-κB signaling pathway.

## Claims

1. The adrenoceptor beta 1 (ADRB 1) active inhibitor acebutolol hydrochloride (ACE) for use in a method for treating psoriasis.

2. The ADRB1 active inhibitor ACE for use according to claim 1, wherein the ADRB1 active inhibitor ACE inhibits the abnormal activation of skin sympathetic nerves.

3. The ADRB1 active inhibitor ACE for use according to claim 2, wherein the ADRB1 active inhibitor ACE inhibits the increase of norepinephrine caused by the abnormal activation of the skin sympathetic nerves.

4. The ADRB1 active inhibitor ACE for use according to claim 1, wherein the ADRB1 active inhibitor ACE inhibits the secretion of interleukin 17 (IL-17) by γδT cells.

## Patentansprüche

1. Adrenozeptor-beta-1(ADRB1)-Aktivhemmer Acebutololhydrochlorid (ACE) zur Verwendung in einem Verfahren zur Behandlung von Psoriasis.

2. ADRB1-Aktivhemmer ACE zur Verwendung nach Anspruch 1, wobei der ADRB1-Aktivhemmer ACE die abnormale Aktivierung der Hautsympathikusnerven hemmt.

3. ADRB1-Aktivhemmer ACE zur Verwendung nach Anspruch 2, wobei der ADRB1-Aktivhemmer ACE den durch die abnormale Aktivierung der Hautsympathikusnerven verursachten Anstieg von Noradrenalin hemmt.

4. ADRB1-Aktivhemmer ACE zur Verwendung nach Anspruch 1, wobei der ADRB1-Aktivhemmer ACE die Sekretion von Interleukin 17 (IL-17) durch γδ-T-Zellen hemmt.

## Revendications

1. Inhibiteur actif du récepteur bêta-1 adrénergique (ADRB1), le chlorhydrate d'acébutolol (ACE), à utiliser dans une méthode de traitement du psoriasis.

2. Inhibiteur actif de l'ADRB1, l'ACE, à utiliser selon la revendication 1, l'inhibiteur actif de l'ADRB1, l'ACE, inhibant l'activation anormale des nerfs sympathiques cutanés.

3. Inhibiteur actif de l'ADRB1, l'ACE, à utiliser selon la revendication 2, l'inhibiteur actif de l'ADRB1, l'ACE, inhibant l'augmentation de la noradrénaline provoquée par l'activation anormale des nerfs sympathiques cutanés.

4. Inhibiteur actif de l'ADRB1, l'ACE, à utiliser selon la revendication 1, l'inhibiteur actif de l'ADRB1, l'ACE, inhibant la sécrétion d'interleukine 17 (IL-17) par les cellules γδT.
